# EUROPEAN PATENT APPLICATION

(11) **EP 2 248 810 A1**
(43) Date of publication of application: **10.11.2010**
(21) Application number: 09713617.0
(22) Date of filing: 13.02.2009
(51) Int. Cl.: C07D 401/12, C07D 211/58, C07D 213/74, C07D 213/75, C07D 213/81

(54) **METHOD FOR PRODUCING PHENOXYPYRIDINE DERIVATIVE**

(30) Priority: 18.02.2008 JP 2008036161
(71) Applicant: Eisai R&D Management Co., Ltd., Tokyo 112-8088 (JP)
(72) Inventor: NAGAI, Mitsuo, Tsukuba-shi Ibaraki 300-2635 (JP)
(74) Representative: HOFFMANN EITLE
(86) International application number: PCT/JP2009/052401
(87) International publication number: WO 2009/104520

(57) **Abstract**

A method for producing a compound represented by the formula (I) 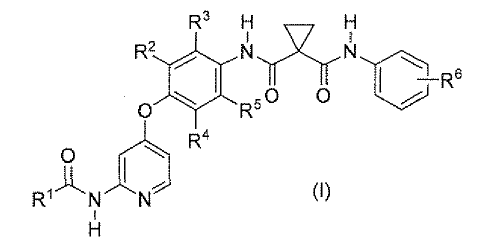 the method comprising reacting in the presence of a condensation agent a compound represented by the formula (II) or a salt thereof 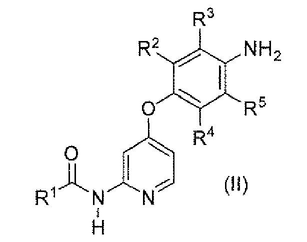 with a compound represented by the formula (III) 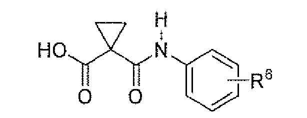 wherein R¹ represents a 4-(4-methylpiperadin-1-yl) group or 3-hydroxyazetidin-1-ml group, R², R³, R⁴ and R⁵ may be the same or different and each represents a hydrogen atom or a fluorine atom and R⁶ represents a hydrogen atom or a fluorine atom.

## Description

### Technical Field

The present invention relates to a method for producing a phenoxypyridine derivatives (hereafter referred to as "the present compound") useful as an anti-tumor agent and an inhibitor for cancer metastasis having inhibitory activity against hepatocyte growth factor receptor (hereafter referred to as "HGFR"), anti-tumor activity, inhibitory activity against angiogenesis, inhibitory activity against cancer metastasis or the like, and to production intermediates in the production method.

### Background Art

Patent Literature 1 discloses a phenoxypyridine derivative having inhibitory activity against HGFR and being useful as an anti-tumor anent, inhibitor for angiogenesis or inhibitor for cancer metastasis and a method for producing thereof

### Citation List

### Patent Literature

Patent Literature 1: WO 2007/023768

### Summary of Invention

### Technical Problem

An object of the invention is to find an improved method for producing the phenoxypyridine derivative useful as an anti-tumor agent, inhibitor for angiogenesis, inhibitor for cancer metastasis disclosed in Patent Literature 1 as well as a production intermediate in the production method.

### Solution to Problem

As a result of diligent studies in view of the above situation, the inventors have found a method for producing a phenoxypyridine derivative suitable for industrial large scale synthesis and a production intermediate in the production method, and completed the invention.

More specifically, the present invention provides the following [1] to [4].
[1] A method for producing a compound represented by the formula (I) 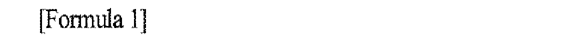 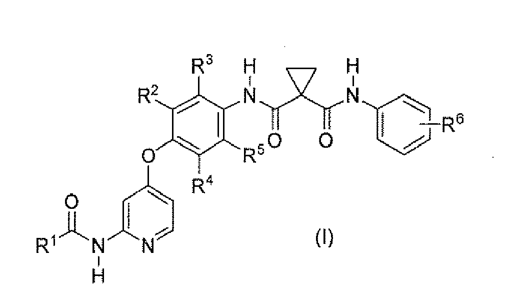 wherein R¹ represents a 4-(4-methylpiperadin-1-yl)piperidin-1-yl group or a 3-hydroxyazetidin-1-yl group, R², R³, R⁴ and R⁵ may be the same or different and each represents a hydrogen atom or a fluorine atom, provided that two or three of R², R³, R⁴ and R⁵ are a hydrogen atom, and R⁶ represents a hydrogen atom or a fluorine atom,
   the method comprising:
   1) protecting an amino group of a compound represented by the formula (IX) 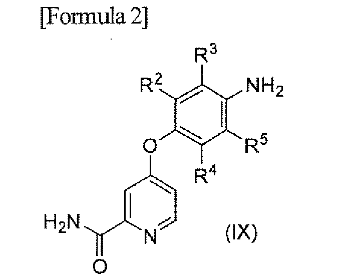 wherein R², R³, R⁴ and R⁵ have the same definitions as defined above
      to produce a compound represented by the formula (VIII) 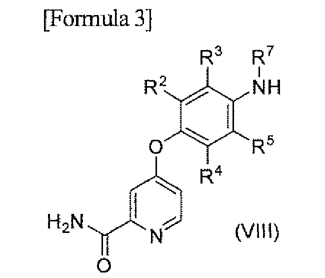 wherein R², R³, R⁴ and R⁵ have the same definitions as defined above and R⁷ represents a protection group for an amino group,
   2) subsequently reacting the compound represented by the formula (VIII) with a Hofmann rearrangement agent to produce a compound represented by the formula (VI) 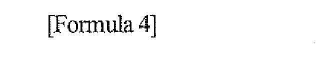 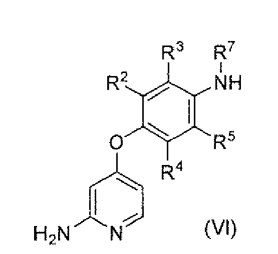 wherein R², R³, R⁴, R⁵ and R⁷ have the same definitions as defined above,
   3) subsequently reacting the compound represented by the formula (VI) with a compound represented by the formula (VII) 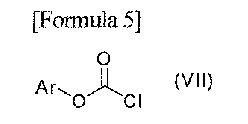 wherein Ar represents a phenyl group optionally substituted with one or two substituents selected from a halogen atom, a methyl group, a methoxy group, a nitro group, a cyano group and a trifluoromethyl group
      to produce a compound represented by the formula (V) 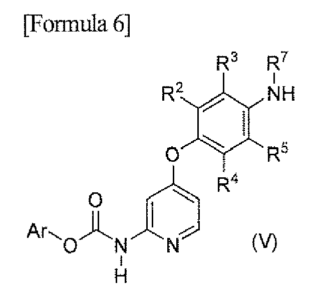 wherein R², R³, R⁴, R⁵, R⁷ and Ar have the same definitions as defined above,
   4) subsequently reacting the compound represented by the formula (V) with an amine selected from 1-methyl-4-(piperidin-4-yl)piperazine and 3-hydroxyazetidine or a salt thereof to produce a compound represented by the formula (IV) or a salt thereof 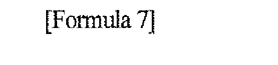 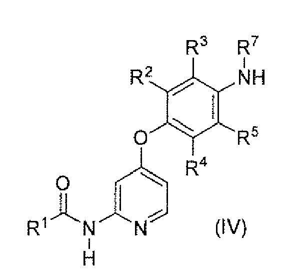 wherein R¹, R², R³, R⁴, R⁵ and R⁷ have the same definitions as defined above,
   5) subsequently deprotecting the amino group of the compound represented by the formula (IV) or a salt thereof to produce a compound represented by the formula (II) or a salt thereof 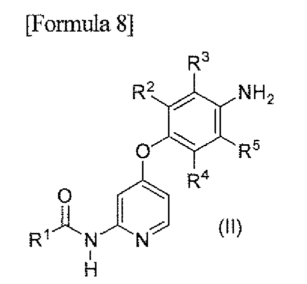 wherein R¹, R², R³, R⁴ and R⁵ have the same definitions as defined above, and
   6) reacting the compound represented by the formula (II) or a salt thereof with a compound represented by the formula (III) in the presence of a condensation agent, 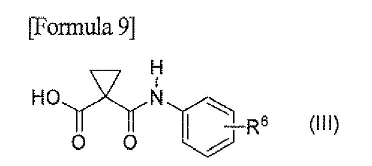 wherein R⁶ has the same definitions as defined above to produce the compound represented by the formula (I);
[2] the production method according to [1], wherein the Hofmann rearrangement agent is iodobenzene diacetate or iodobenzene bis(trifluoroacetate);
[3] the production method according to [1], wherein the condensation agent is O-(7-azabenzotriazol-1-yl)-N,N,N',N'-tetramethyluronium hexafluorophosphate (HATU); and
[4] the production method according to [1], wherein R⁷ is a t-butoxycarbonyl group, a trifluoroacetyl group or a trichloroacetyl group.

### Advantageous Effects of Invention

The present invention can provide a method for producing a phenoxypyridine derivative suitable for industrial large scale synthesis, having an HGFR inhibitory activity and having anti-tumor activity, inhibitory activity against angiogenesis, inhibitory activity against cancer metastasis, or the like. The present invention also provides a production intermediate useful in the above production method.

### Description of Embodiments

The symbols and terms as used herein will be defined and the present invention will be described in details below.

The structural formulas for the compounds throughout the present specification may represent only certain isomeric form for the sake of convenience, but the invention encompasses all isomers such as geometric isomers, optical isomers based on asymmetric carbons, stereoisomers, tautomers, and mixtures of those isomers which occur due to the structures of the compounds, without being limited to any of the formulas shown for the sake of convenience and may be either one of isomers or a mixture thereof. The compounds of the invention therefore may sometimes contain asymmetric carbons in the molecular and an optically active or racemic form may be present, but the present invention is not limited to either one but includes both of them. There are also no restrictions when polymorphic crystalline forms thereof exist, and the compounds may be in one crystalline form or a mixture of different crystalline forms. Further, anhydrates and hydrates of the compounds of the invention are also included.

The "salt" is not particularly limited so long as a salt can be formed with the compound according to the present invention and includes, for example, a salt with an inorganic acid, a salt with an organic acid, a salt with an inorganic base, a salt with an organic base, a salt with an acidic or basic amino acid or the like.

The preferable salt with an inorganic acid includes, for example, a salt with hydrochloric acid, hydrobromic acid, sulfuric acid, nitric acid, phosphoric acid or the like. The preferable salt with an organic acid includes, for example, a salt with acetic acid, succinic acid, fumaric acid, maleic acid, tartaric acid, citric acid, lactic acid, stearic acid, benzoic acid, methanesulfonic acid, ethanesulfonic acid, p-toluenesulfonic acid or the like.

The preferable salt with an inorganic base includes, for example, an alkali metal salt such as sodium salt and potassium salt, an alkali earth metal salt such as calcium salt and magnesium salt, aluminum salt, ammonium salt or the like. The preferable salt with an organic base includes, for example, a salt with diethylamine, diethanolamine, meglumine, N,N-dibenzylethylenediamine or the like.

The preferable salt with an acidic amino acid includes, for example, a salt with aspartic acid, glutamic acid or the like. The preferable salt with a basic amino acid includes, for example, a salt with arginine, lysine, ornithine or the like.

The "condensation agent" in the above [1] represents 4-(4,6-dimethoxy[1.3.5]triazin-2-yl)-4-methylmorpholinium chloride hydrate, 2-chloro-4,6-dimethoxy-1,3,5-triazine, 2,4,6-trichloro-1,3,5-triazine, dicyclohexyl carbodiimide (DCC), 1-ethyl-3,(3'-dimethylaminopropyl)carbodiimide HCl salt (EDC or WSC HCl), O-(7-Azabenzotriazol-1-yl)-N,N,N',N'-tetramethyluronium hexafluorophosphate (HATU), O-(1H-benzotiazol-1-yl)-N,N,N',N'-tetramethyluronium hexafluorophosphate (HBTU), O-(1H-benzotiazol-1-yl)-N,N,N',N'-tetramethyluronium tetrafluoroborate (TBTU), (Benzotriazol-1-yloxy)tris(dimethylamino)phosphonium hexafluorophosphate (BOP reagent) or the like and O-(7-Azabenzotriazol-1-yl)-N,N,N',N'-tetramethyluronium hexafluorophosphate (HATU) is preferable.

The "Hofmann rearrangement agent" in the above [1] represents iodobenzene diacetate, iodobenzene bis(trifluoroacetate), sodium hypochlorite, potassium hypobromite, bromine, iodine or the like, and iodobenzene diacetate or iodobenzene bis(trifluoroacetate) is preferable.

The respective substituents of the compound represented by the above formulas (I) to (IX) according to the present invention will be described below.

### [The definition of R¹]

R¹ represents a 4-(4-methylpiperazin-1-yl)piperidin-1-yl group or a 3-hydroxyazetidin-1-yl group.

### [The definition of R², R³, R⁴ and R⁵]

R², R³, R⁴ and R⁵ may be the same or different and each represents a hydrogen atom or a fluorine atom, provided that two or three of R², R³, R⁴ and R⁵ are a hydrogen atom.
Preferable examples of the group represented by the formula: 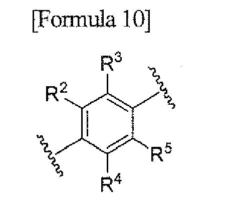 include a group represented by the formula:

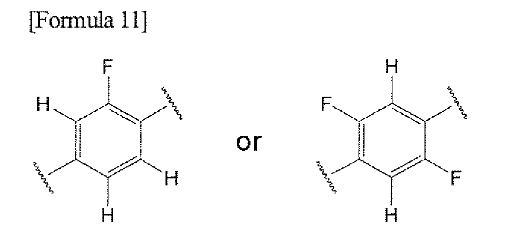

### [The definition of R⁶]

R⁶ represents a hydrogen atom or a fluorine atom.
Preferable examples of R⁶ include a fluorine atom.

### [The definition of R⁷]

R⁷ represents a protection group of the amino group conventionally used, and specific examples include a t-butoxycarbonyl group, a benzyloxycarbonyl group, a 9-fluorenyl methoxycarbonyl group, a vinyloxycarbonyl group, a trifluoroacetyl group, a trichloroacetyl group, an acetyl group, a formyl group and the like.
Preferable examples of R⁷ include a t-butoxycarbonyl group, a trifluoroacetyl group or trichloroacetyl group.

### [The definition of Ar]

Ar represents a phenyl group optionally substituted with one or two substituents selected from a halogen atom, a methyl group, a methoxy group, a nitro group, a cyano group and a trifluoromethyl group.
Preferable examples ofAr include a phenyl group.

The production method according to the present invention is described in details below. 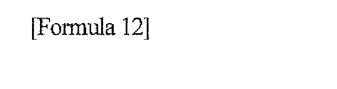 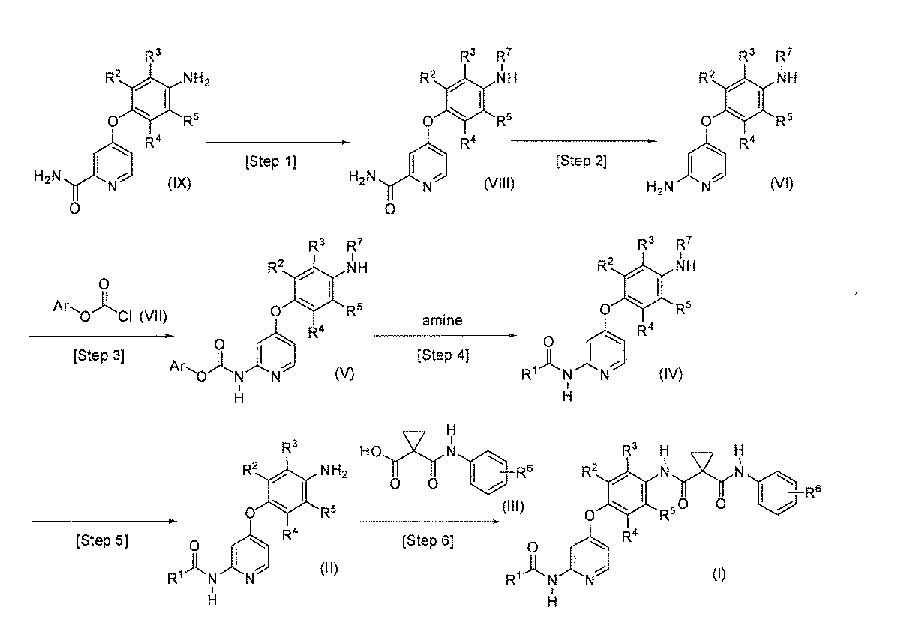 wherein each symbol has the same definition as defined above.

### [Step 1]

This is a step of producing a compound (VIII) by protecting the amino group of a compound (IX).
As the compound (IX) may be used the compounds described in Examples below, publicly known compounds, commercially available compounds or compounds easily produced by methods those skilled in the art usually carry out from commercially available compounds.
The protection agent for an amino group refers to di-t-butylcarbonate, 2-t-butoxycarbonyloimino-2-phenylacetonitrile, t-butyl azidoformate, benzyloxycarbonyl chloride, 9-fluorenylmethyl chloroformate, vinyl chloroformate, trifluoroacetic anhydride, trichloroacetic anhydride, acetic anhydride, formic acid and the like, and di-t-butylcarbonate, trifluoroacetic anhydride, trichloroacetic anhydride are preferable.
The solvent used for this step is not particularly limited so long as it dissolves starting materials to some extent and does not inhibit the reaction, and includes, for example, ether solvents such as tetrahydrofuran, 1,2-dimethoxyethane, 1,4-dioxane, tert-butyl methyl ether, cyclopentyl methyl ether, diethyl ether, diisopropyl ether, dibutyl ether and dicyclopentyl ether; N,N-dimethylformamide; N-methyl-2-pyrrolidone; or a mixed solvent thereof or the like, and tetrahydrofuran, N,N-dimethylformamide and N-methyl-2-pyrrolidone are preferable.
The reaction temperature will generally differ depending on the starting materials, the solvent and the other reagents used in the reaction and it is preferably 0°C to 50°C (internal temperature of the reaction vessel) and more preferably 0°C to 30°C (internal temperature of the reaction vessel).
The reaction time will generally differ depending on the starting materials, the solvent, the other reagents used in the reaction and the reaction temperature, and stirring the reaction mixture at the above reaction temperature for 1 to 48 hours after the addition of the reagents is preferable and stirring for 4 to 24 hours is more preferable.
The protection agent of the amino group can be used in an amount of 1.0- to 3.0-fold molar equivalent with respect to compound (IX), and preferably it is used in an amount of 1.0- to 1.3-fold molar equivalent.

### [Step 2]

This is a step of producing a compound (VI) by reacting the compound (VIII) with a Hofmann rearrangement agent.
The solvent used for this step is not particularly limited so long as it dissolves starting materials to some extent and does not inhibit the reaction, and include, for example, N,N-dimethylformamide, N,N-dimethylacetamide, dimethyl sulfoxide, N-methyl-2-pyrrolidone or the like, and N,N-dimethylformamide and N-methyl-2-pyrrolidone are preferable.
The "Hofmann rearrangement agent" represents iodobenzene diacetate, iodobenzene bis(triffuoroacetate), sodium hypochlorite, potassium hypobromite, bromine, iodine or the like, and iodobenzene diacetate and iodobenzene bis(trifluoroacetate) are preferable.
The reaction temperature will generally differ depending on the starting materials, the solvent and the other reagents used in the reaction, and it is preferably -10°C to 50°C (internal temperature of the reaction vessel) and more preferably 20°C to 30°C (internal temperature of the reaction vessel).
The reaction time will generally differ depending on the starting materials, the solvent, the other reagents used in the reaction and the reaction temperature, and stirring the reaction mixture at the above reaction temperature for 1 to 24 hours after the addition of the reagents is preferable and stirring for 3 to 5 hours is more preferable.
The Hofmann rearrangement agent can be used in an amount of 1.0- to 3.0-fold molar equivalent with respect to the compound (VIII), and preferably it is used in an amount of 1.0- to 1.2-fold molar equivalent.

### [Step 3]

This is a step of producing a compound (V) by reacting the compound (VI) with compound (VII) in the presence of a base.
As the compound (VII) may be used publicly known compounds, commercially available compounds or compounds easily produced by methods those skilled in the art usually carry out from commercially available compounds.
The solvent used for this step is not particularly limited so long as it dissolves starting materials to some extent and does not inhibit the reaction, and includes, for example, ether solvents such as tetrahydrofuran, 1,2-dimethoxyethane, tert-butyl methyl ether, cyclopentyl methyl ether, diethyl ether, diisopropyl ether, dibutyl ether and dicyclopentyl ether; aromatic hydrocarbon solvents such as benzene and toluene; aliphatic hydrocarbon solvents such as heptane and hexane; acetonitrile; or a mixed solvent thereof or the like, and a mixed solvent of tetrahydrofuran and acetonitrile is preferable.
The base represents pyridine, triethylamine, diisopropylethylamine, potassium carbonate, sodium carbonate or the like, and pyridine is preferable.
The reaction temperature will generally differ depending on the starting materials, the solvent and the other reagents used in the reaction, and it is preferably -10°C to 50°C (internal temperature of the reaction vessel) and more preferably 0°C to 30°C (internal temperature of the reaction vessel).
The reaction time will generally differ depending on the starting materials, the solvent, the other reagents used in the reaction and the reaction temperature, and stirring the reaction mixture at the above reaction temperature for 1 to 24 hours after the addition of the reagents is preferable and stirring for 2 to 5 hours is more preferable.
The compound (VII) can be used in an amount of 1.0- to 3.0-fold molar equivalent with respect to the compound (VI), and preferably it is used in an amount of 1.1-to 2.0-fold molar equivalent.
The base can be used in an amount of 1.0- to 3.0-fold molar equivalent with respect to the compound (VI), and preferably it is used in an amount of 1.1- to 2.0-fold molar equivalent.

### [Step 4]

This is a step of producing a compound (IV) or a salt thereof by reacting the compound (V) with an appropriate amine (or a salt thereof) in the presence or absence of a base.
As the amine may be used amines selected from 1-methyl-4-(piperidin-4-yl)piperazine and 3-hydroxyazetidine.
The solvent used for this step is not particularly limited so long as it dissolves starting materials to some extent and does not inhibit the reaction, and includes, for example, N,N-dimethylformamide, N-methyl-2-pyrrolidone, N,N-dimethylacetamide, dimethyl sulfoxide or the like, and N-methyl-2-pyrrolidone is preferable.
The base represents potassium carbonate, sodium carbonate, pyridine, triethylamine, diisopropylethylamine or the like, and potassium carbonate is preferable.
The reaction temperature will generally differ depending on the starting materials, the solvent and the other reagents used in the reaction, and it is preferably 10°C to 100°C (internal temperature of the reaction vessel) and more preferably 20°C to 50° (internal temperature of the reaction vessel).
The reaction time will generally differ depending on the starting materials, the solvent, the other reagents used in the reaction and the reaction temperature, and stirring the reaction mixture at the above reaction temperature for 1 to 24 hours after the addition of the reagents is preferable and stirring for 1 to 4 hours is more preferable.
The amine (or a salt thereof) can be used in an amount of 1.0- to 3.0-fold molar equivalent with respect to the compound (V), and preferably it is used in an amount of 1.1-to 1.3-fold molar equivalent.
The base can be used in an amount of 1.0- to 3.0-fold molar equivalent with respect to the compound (V), and preferably it is used in an amount of 1.1- to 1.3-fold molar equivalent.

### [Step 5]

This is a step of producing a compound (II) or a salt thereof by deprotecting the protection group of the amino group of the compound (IV) or a salt thereof

### (1) In the case of hydrolysis

The compound (II) or a salt thereof can be produced by the hydrolysis of the compound (IV) or a salt thereof in the presence of an acid or a base.
The solvent used for this step is not particularly limited so long as it dissolves starting materials to some extent and does not inhibit the reaction, and includes, for example, alcohol solvents such as methanol, ethanol, propanol and butanol; ether solvents such as tetrahydrofuran, 1,2-dimethoxyethane, tert-butyl methyl ether, cyclopentyl methyl ether, diethyl ether, diisopropyl ether, dibutyl ether and dicyclopentyl ether; water; or a mixed solvent thereof or the like, and a mixed solvent of water and methanol, ethanol or tetrahydrofuran is preferable.
The acid represents hydrochloric acid, trifluoroacetic acid, hydrobromic acid, acetic acid, p-toluenesulfonic acid, methanesulfonic acid or the like, and hydrochloric acid is preferable.
The base represents sodium hydroxide, potassium hydroxide, potassium carbonate, sodium carbonate, sodium hydrogencarbonate, ammonia, dimethylamine or the like.
The reaction temperature will generally differ depending on the starting materials, the solvent and the other reagents used in the reaction, and it is preferably 0°C to 80°C (internal temperature of the reaction vessel) and more preferably 30°C to 50°C (internal temperature of the reaction vessel).
The reaction time will generally differ depending on the starting materials, the solvent, the other reagents used in the reaction and the reaction temperature, and stirring the reaction mixture at the above reaction temperature for 1 to 24 hours after the addition of the reagents is preferable and stirring for 2 to 5 hours is more preferable.
The acid can be used in an amount of 1.0- to 5.0-fold molar equivalent with respect to the compound (IV), and preferably it is used in an amount of 1.0- to 2.0-fold molar equivalent.
The base can be used in an amount of 1.0- to 5.0-fold molar equivalent with respect to the compound (IV), and preferably it is used in an amount of 1.0- to 2.0-fold molar equivalent.

### (2) In the case of catalytic hydrogenation

This is a step of producing a compound (II) or a salt thereof by the catalytic hydrogenation of the compound (IV) or a salt thereof in the presence of a reduction catalyst under a hydrogen atmosphere.
The solvent used for this step is not particularly limited so long as it dissolves starting materials to some extent and does not inhibit the reaction, and includes, for example, alcohol solvents such as methanol, ethanol, propanol and butanol; ether solvents such as tetrahydrofuran, 1,2-dimethoxyethane, tert-butyl methyl ether, cyclopentyl methyl ether, diethyl ether, diisopropyl ether, dibutyl ether and dicyclopentyl ether; N,N-dimethylformamide; N-methyl-2-pyrrolidone; formic acid; water; or a mixed solvent thereof, and a mixed solvent of water, methanol and tetrahydrofuran, a mixed solvent of water, ethanol and tetrahydrofuran, or a mixed solvent of water and ethanol is preferable.
The reduction catalyst represents palladium on carbon, palladium hydroxide, platinum oxide, Raney nickel or the like, and palladium on carbon is preferable.
This step can be carried out under a hydrogen atmosphere at 0.1 MPa (ordinary pressure) to 1.0 MPa, and more preferably under a hydrogen atmosphere at 0.1 MPa to 0.3 MPa.
When formic acid or a mixed solvent containing formic acid is used as a solvent for this step, this step can be carried out without using hydrogen gas.
The reaction temperature will generally differ depending on the starting materials, the solvent and the other reagents used in the reaction, and it is preferably 0°C to 50°C (internal temperature of the reaction vessel) and more preferably 20°C to 30°C (internal temperature of the reaction vessel).
The reaction time will generally differ depending on the starting materials, the solvent, the other reagents used in the reaction and the reaction temperature, and stirring the reaction mixture at the above reaction temperature for 1 to 48 hours after the addition of the reagents is preferable and stirring for 3 to 18 hours is more preferable.
The reduction catalyst can be used in an amount of 0.1- to 5.0-fold molar equivalent with respect to the compound (IV), and preferably it is used in an amount of 0.5-to 1.5-fold molar equivalent.

### (3) In the case of reduction

This is a step of producing a compound (II) or a salt thereof by reducing the compound (IV) or a salt thereof in the presence of a reducing agent.
The solvent used for this step is not particularly limited so long as it dissolves starting materials to some extent and does not inhibit the reaction, and includes, for example, alcohol solvents such as methanol, ethanol, 1-propartol, 2-propanol and butanol; water; or a mixed solvent thereof, and methanol and ethanol are preferable.
The reducing agent represents sodium borohydride, lithium borohydride, sodium cyanoborohydride, sodium triacetoxyborohydride, lithium bis(ethoxymethoxy)aluminum hydride, lithium aluminum hydride, or the like.
The reaction temperature will generally differ depending on the starting materials, the solvent and the other reagents used in the reaction, and it is preferably 0°C to 50°C (internal temperature of the reaction vessel) and more preferably 0°C to 30°C (internal temperature of the reaction vessel).
The reaction time will generally differ depending on the starting materials, the solvent, the other reagents used in the reaction and the reaction temperature, and stirring the reaction mixture at the above reaction temperature for 1 to 48 hours after the addition of the reagents is preferable and stirring for 2 to 6 hours is more preferable.
The reducing agent can be used in an amount of 1.0- to 30-fold molar equivalent with respect to the compound (IV), and preferably it is used in an amount of 1.0- to 20-fold molar equivalent.

### [Step 6]

This is a step of producing a compound (I) by reacting the compound (II) or a salt thereof with a compound (III) in the presence of a condensation agent and in the presence or absence of abase.
As the compound (III) may be used publicly known compounds, commercially available compounds or compounds easily produced by methods those skilled in the art usually carry out.
The solvent used for this step is not particularly limited so long as it dissolves starting materials to some extent and does not inhibit the reaction, and includes, for example, ether solvents such as tetrahydrofuran, 1,2-dimethoxyethane, tert-butyl methyl ether, cyclopentyl methyl ether, diethyl ether, diisopropyl ether, dibutyl ether and dicyclopentyl ether; alcohol solvents such as ethanol, 1-propanol, 2-propanol; N,N-dimethylformamide; N-methyl-2-pyrrolidone; N,N-dimethylacetamide; or a mixed solvent thereof, and N,N-dimethylformamide is preferable.
The condensation agent refers to 4-(4,6-dimethoxy[1.3.5]triazin-2-yl)-4-methylmorpholinium chloride hydrate, 2-chloro-4,6-dimethoxy-1,3,5-triazine, 2,4,6-trichloro-1,3,5-triazine, dicyclohexyl carbodiimide (DCC), 1-ethyl-3,(3'-dimethylaminopropyl)carbodiimide HCl salt (EDC or WSC HCl), O-(7-Azabenzotriazol-1-yl)-N,N,N',N'-tetramethyluronium hexafluorophosphate (HATU), O-(1H-benzotiazol-1-yl)-N,N,N',N'-tetramethyluronium hexafluorophosphate (HBTU), O-(1H-benzotiazol-1-yl)-N,N,N',N'-tetramethyluronium tetrafluoroborate (TBTU), (Benzotriazol-1-yloxy)tris(dimemylamino)phosphonium hexafluorophosphate (BOP reagent) or the like and O-(7-Azabenzotiazol-1-yl)-N,N,N',N'-tetramethyluronium hexafluorophosphate (HATU) is preferable.
The base represents N-methylmorpholine, pyridine, triethylamine, diisopropylethylamine, 1-methylimidazol, potassium carbonate, sodium carbonate or the like, and triethylamine is preferable.
The reaction temperature will generally differ depending on the starting materials, the solvent and the other reagents used in the reaction, and it is preferably -10°C to 50°C (internal temperature of the reaction vessel) and more preferably 20°C to 30°C (internal temperature of the reaction vessel).
The reaction time will generally differ depending on the starting materials, the solvent, the other reagents used in the reaction and the reaction temperature, and stirring the reaction mixture at the above reaction temperature for 1 to 48 hours after the addition of the reagents is preferable and stirring for 3 to 18 hours is more preferable.
The compound (III) can be used in an amount of 1.0- to 3.0-fold molar equivalent with respect to the compound (II), and preferably it is used in an amount of 1.0- to 2.0-fold molar equivalent.
The condensation agent can be used in an amount of 1.0- to 3.0-fold molar equivalent with respect to the compound (II), and preferably it is used in an amount of 1.0-to 2.0-fold molar equivalent
The base can be used in an amount of 1.0- to 10-fold molar equivalent with respect to the compound (II), and preferably it is used in an amount of 2.0- to 4.0-fold molar equivalent.

### [Example]

Examples are illustrated below for the purpose of the easy understanding of the present invention, but the present invention is not limited to these Examples.

### Example A-1: [4-(2-Carbamoylpyridin-4-yloxy)-2-fluorophenyl]carbamic acid t-butyl ester

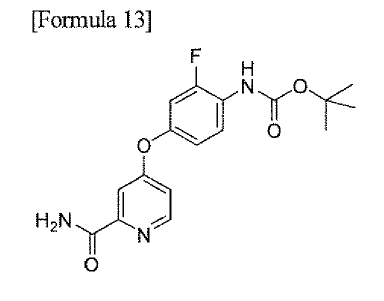

After di-t-butyldicarbonate (212 mg) was added at room temperature to a solution of 4-(4-amino-3-fluorophenoxy)pyridine-2-carboxamide (200 mg) in N,N-dimethylformamide (1 ml), 1,4-dioxane (1 ml) and 2-methyl-2-propanol (1 ml), the mixture was heated to 65°C and stirred for 14 hours. Di-t-butyldicarbonate (212 mg) was further added thereto and stirred for 6.5 hours at 65°C. The reaction mixture was diluted using ethyl acetate (2 ml), the mixture was washed with a saturated aqueous solution of sodium hydrogen carbonate and partitioned. Subsequently, the organic layer was washed with a 5% aqueous solution of sodium chloride and dried over anhydrous magnesium sulfate. The solvent was distilled off under reduced pressure to give the residue. The residue was purified by silica gel column chromatography (eluent; 50 to 60/50 to 40 = ethyl acetate/n-heptane) to give the titled compound (176 mg).
¹H-NMR Spectrum (DMSO-d₆) δ (ppm): 1.47 (9H, s), 7.04 (1H, dd, J=2.8, 9.2 Hz), 7.19 (1H, dd, J=2.4, 5.6 Hz), 7.27 (1H, dd, J=2.8, 11.2 Hz), 7.40 (1H, d, J=2.8 Hz), 7.64-7.75 (2H, m), 8.13 (1H, brs), 8.53 (1H, d, J=5.6 Hz), 9.07 (1H, brs)

### Example A-2: [4-(2-aminopyridin-4-yloxy)-2-fluorophenyl]carbamic acid t-butyl ester

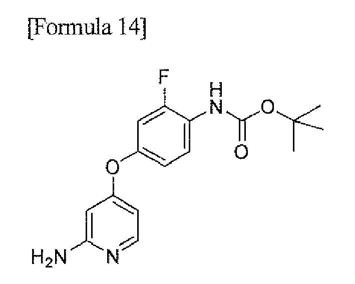

Iodobenzene diacetate (604 mg) was added with stirring at room temperature to a solution of [4-(2-carbamoylpyridin-4-yloxy)-2-fluorophenyl]carbamic acid t-butyl ester (244 mg) in N,N-dimethylformamide (2.5 mL), water (63 L) and piridine (0.34 ml), and the mixture was stirred for 42 hours. Ethyl acetate (5 mL) was added to the reaction mixture, a 1N sodium hydroxide aqueous solution (5 ml) was added to quench the reaction, and the layers were separated. The organic layer was washed with a 5% aqueous solution of sodium chloride, dried over anhydrous magnesium sulfate, filtered and concentrated. The residue was purified by silica gel column chromatography (Fuji silysia NH, eluent; 40/60 = ethyl acetate/n-heptane) to give the title compound (91 mg).
¹H-NMR Spectrum (DMSO-d₆) δ (ppm): 1.46 (9H, s), 5.83 (1H, d, J=2.4 Hz), 5.92-5.98 (2H, m), 6.15 (1H, dd, J=2.4, 6.0 Hz), 6.93 (1H, d, J=8.8 Hz), 7.56 (1H, dd, J=2.4, 11.2 Hz), 7.59 (1H, t, J=9.2 Hz), 7.81 (1H, d, J=5.6 Hz), 9.01 (1H, brs).

### Example A-3: [2-fluoro-4-(2-{[4-(4-methylpiperazin-1-yl)piperidine-1-carbonyl]amino}pyridin-4-yloxy)phenylcarbamic acid t-butyl ester

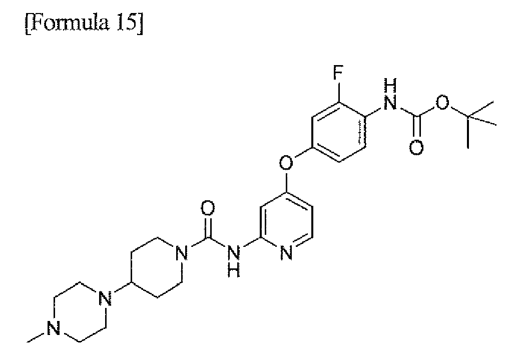

N,N-diisopropylethylamine (109 µl) and phenyl chloroformate (98 mg) were added to a solution of [4-(2-aminopyridin-4-yloxy)-2-fluorophenyl]carbamic acid t-butyl ester (91 mg) in tetrahydrofuran (1 ml) with stirring and cooling on ice and the mixture was stirred for 60 minutes. After the solvent was concentrated under reduced pressure, N,N dimethylformamide (1 ml) was added to the residue, 1-methyl-4-(piperidin-4-yl) piperazine (125 ml) was added thereto and the mixture was stirred at room temperature for 40.5 hours. Ethyl acetate (10 ml) and a saturated aqueous solution of sodium hydrogen carbonate (2 ml) were added to the reaction mixture and the mixture was partitioned. The organic layer was washed with a 5% aqueous solution of sodium chloride (3 ml) and dried over anhydrous magnesium sulfate. After filtered, the residue obtained by concentration was purified by silica gel column chromatography (Fuji silysia NH, eluent; 95/5 = ethyl acetate/methanol) to give the title compound (126 mg).
¹H-NMR Spectrum (DMSO-d₆) δ (ppm): 1.20-1.35 (2H, m), 1.47 (9H, s), 1.67-1.76 (2H, m), 2.12 (3H, s), 2.22-2.50 (8H, m), 2.68-2.79 (2H, m), 3.31 (1H, s), 4.06-4.14 (2H, m), 6.55-6.62 (1H, m), 6.75 (1H, d, J=7.6 Hz), 6.88-6.98 (1H, m), 7.36-7.39 (1H, m), 7.58-7.69 (1H, m), 8.12 (1H, d, J=5.6 Hz), 9.03 (1H, brs), 9.20 (1H, brs).

### Example A-4: 4-(4-methylpiperazin-1-yl)piperidine-1-carboxylic acid [4-(4-amino-3-fluorophenoxy)pyridin-2-yl]amide

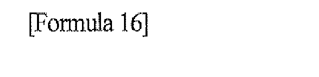

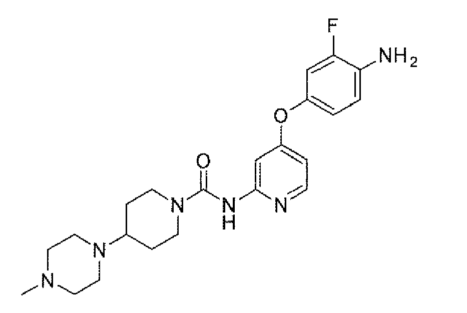

A solution of 4N hydrochloric acid in ethyl acetate (1 g) was added at room temperature to [2-fluoro-4-(2-{[4-(4-methylpiperazin-1-yl)piperidine-1-carbonyl]amino}pyridin-4-yloxy)phenylcarbamic acid t-butyl ester (58 mg) and the mixture was stirred for 2 hours at the same temperature. Ethyl acetate (10 ml) was added to the reaction mixture, and a 5N sodium hydroxide aqueous solution was added until the mixture became alkaline. The separated organic layer was washed with a 5% aqueous solution of sodium chloride and dried over anhydrous magnesium sulfate. After filtered, the solvent was concentrated to give the titled compound (33 mg).
¹H-NMR Spectrum (DMSO-d₆) δ (ppm): 1.20-1.34 (2H, m), 1.68-1.74 (2H, m), 2.12 (3H, s), 2.20-2.55 (8H, m), 2.72 (2H, d, J=11.6 Hz), 3.32 (1H, brs), 4.09 (2H, d, J=13.2 Hz), 5.12-5.15 (2H, m), 6.50 (1H, dd, J=2.4, 6.0 Hz), 6.72 (1H, dd, J=2.4, 8.4 Hz), 6.81 (1H, t, J=8.8 Hz), 6.92 (1H, dd, J=2.4, 12.0 Hz), 7.31 (1H, d, J=2.0 Hz), 8.05 (1H, d, J=5.6 Hz), 9.10 (1H, s).

### Example A-5: N-(2-fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide

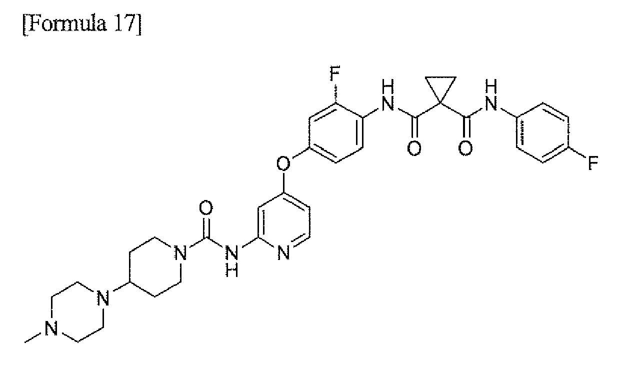

Triethylamine (71 mg) and O-(7-Azabenzotriazol-1-yl)-N,N,N',N'-tetramethyluronium hexafluorophosphate (HATU) (222 mg) were added at room temperature to a solution of 4-(4-methylpiperazin-1-yl)piperidine-1-carboxylic acid [4-(4-amino-3-fluorophenoxy)pyridin-2-yl]amide (100 mg) and 1-(4-fluorophenylcarbamoyl)cyclopropanecarboxylic acid (78 mg) in N,N-dimethylformamide (1 ml) and the mixture was stirred for 21 hours at room temperature. A 1N sodium hydroxide aqueous solution (2 ml) was added to the reaction mixture and extracted with ethyl acetate (15 ml). After partitioned, the organic layer was washed with a 5% aqueous solution of sodium chloride, dried over anhydrous magnesium sulfate and the solvent was distilled off to give the residue. The residue was dissolved in ethyl acetate (3 ml) and extracted with 2N hydrochloric acid (3 ml x 1, 2 ml x 1). The aqueous layer was made into alkaline with a 5N sodium hydroxide aqueous solution (5.5 ml). The mixture was extracted with ethyl acetate, dried over anhydrous magnesium sulfate and the solvent was distilled off to give the titled compound (87 mg).
¹H-NMR Spectrum (DMSO-d₆) δ (ppm): 1.22-1.33 (2H, m), 1.54-1.63 (4H, m), 1.68-1.78 (2H, m), 2.12 (3H, s), 2.12-2.40 (5H, m), 2.40-2.60 (4H, m), 2.68-2.78 (2H, m), 4.06-4.14 (2H, m), 6.60 (1H, dd, J=2.4 Hz, 5.6 Hz), 7.00 (1H, m), 7.19 (2H, t, J=8 Hz), 7.22 (1H, dd, J=2.4 Hz, 11.2 Hz), 7.40 (1H, s), 7.61 (2H, dd, J=5.2 Hz, 8 Hz), 7.93 (1H, t, J=8.8 Hz), 8.13 (1H, d, J=5.6 Hz), 9.21 (1H, s), 9.90 (1H, brs), 10.55 (1H, brs).

### Example B-1: 4-[3-fluoro-4-(2,2,2-trifluoroacetylamino)phenoxy]pyridine-2-carboxylic acid amide

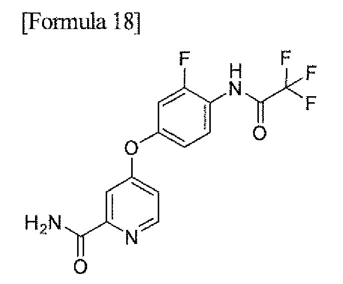

Triethylamine (236 mg) was added to a solution of 4-(4-amino-3-fluorophenoxy)pyridine-2-carboxamide (192 mg) in N-methyl-2-pyrrolidone (3 ml) at room temperature with stirring. After confirming the dissolution, the mixture was cooled with ice, trifluoroacetic anhydride (196 mg) was added thereto, and the mixture was stirred for 5 hours at the same temperature. The reaction mixture was diluted using ethyl acetate (2 ml), the mixture was washed with a saturated aqueous solution of sodium hydrogen carbonate and partitioned. Subsequently, the organic layer was washed with 5% aqueous solution of sodium chloride and dried over the magnesium sulfate. The solvent was distilled off under reduced pressure to give a brown solid product (450 mg). After dissolving the resultant in ethyl acetate (2 ml) with heating at 80°C, n-heptane (2 ml) was added thereto at room temperature and the mixture was stirred for 30 minutes at the same temperature. The crystals were collected by filtration, washed with n-heptane and dried to give the title compound (99 mg).
¹H-NMR Spectrum (DMSO-d₆) δ (ppm): 7.14-7.18 (1H, m), 7.26 (1H, dd, J=2.8, 5.6 Hz), 7.43 (1H, dd, J=2.8, 11.2 Hz), 7.46 (1H, d, J=2.8 Hz), 7.63 (1H, t, J=8.8 Hz), 7.74 (1H, brs), 8.15 (1H, brs), 8.57 (1H, d, J=5.6 Hz), 11.35 (1H, brs).

### Example B-2: N-[4-(2-aminopyridin-4-yloxy)-2-fluorophenyl]-2,2,2-trifluoroacetamide

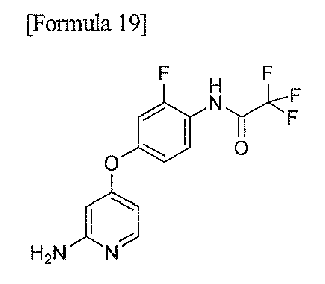

4-[3-Fluoro-4-(2,2,2-trifluoroacetylamino)phenoxy]pyridine-2-carboxylic acid amide (92 mg) as a raw material was reacted and purified in the same manner as in Example A-2 to give the titled compound (47 mg).
¹H-NMR Spectrum (DMSO-d₆) δ (ppm): 5.91 (1H, d, J=2.4 Hz), 5.98-6.06 (2H, m), 6.19 (1H, dd, J=2.0,5.6 Hz), 7.02-7.06 (1H, m), 7.26 (1H, dd, J=2.8, 11.2 Hz), 7.55 (1H, t, J=8.4 Hz), 7.85 (1H, d, J=6.0 Hz), 11.60 (1H, brs).

### Example B-3: 4-(4-(4-methylpiperazin-1-yl)piperidine-1-carboxylic acid [4-[3-fluoro-4-(2,2,2-trifluoroacetylamino)phenoxy]pyridin-2-yl]amide)

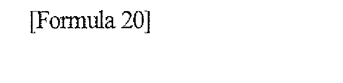

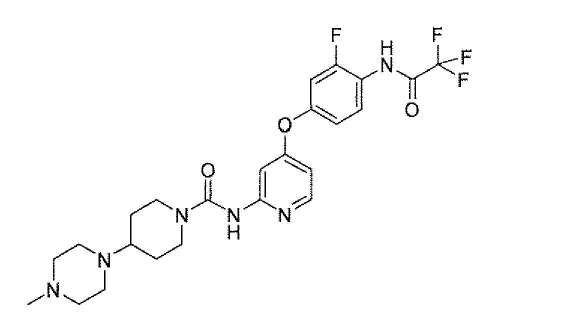

N-[4-(2-Aminopiridin-4-yloxy)-2-fluorophenyl]-2,2,2-trifluoroacetamide (45 mg) as a raw material was reacted and purified in the same manner as in Example A-3 to give the titled compound (45 mg).
¹H-NMR Spectrum (DMSO-d₆) δ (ppm): 1.20-1.35 (2H, m), 1.68-1.77 (2H, m), 2.14 (3H, s), 2.26-2.50 (8H, m), 2.74 (2H, t, J=11.6 Hz), 3.32 (1H, brs), 4.11 (2H, d, J=13.2 Hz), 6.64 (1H, dd, J=2.4, 6.0 Hz), 7.05 (1H, dd, J=2.8, 8.8 Hz), 7.28 (1H, dd, J=2.4, 11.2 Hz), 7.44 (1H, d, J=2.4 Hz), 7.59 (1H, t, J=8.8 Hz), 8.16 (1H, d, J=5.6 Hz), 9.26 (1H, brs).

### Example B-4: 4-(4-methylpiperazin-1-yl)piperidine-1-carboxylic acid [4-(4-amino-3-fluorophenoxy)pyridin-2-yl]amide

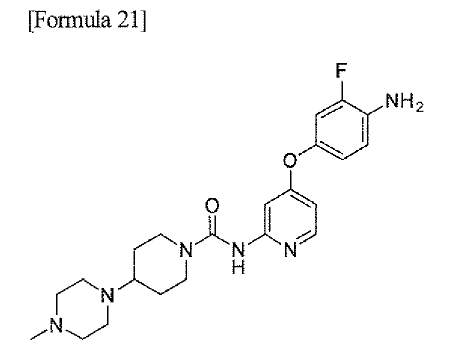

Sodium borohydride (3.2 mg) was added to a solution of 4-(4-(4-methylpiperazin-1-yl)-piperidine-1-carboxylic acid [4-[3-fluoro-4-(2,2,2-trifluoroacetylamino)phenoxy]pyridin-2-yl]amide (22 mg) in ethanol and the mixture was stirred for 15 hours and 40 minutes at room temperature. Subsequently, sodium borohydride (6 mg) was again added at room temperature and the mixture was stirred for 8 hours at room temperature. Further, sodium borohydride (30 mg) was added at room temperature and the mixture was stirred for 20 hours at room temperature. Water (2 ml) was added to the reaction mixture, which was extracted with ethyl acetate (5 ml) and partitioned. After drying the organic layer over magnesium sulfate, the solvent was distilled off under reduced pressure to give the titled compound (11 mg).
The ¹H-NMR data of this sample corresponded to the data described in Example A-4.

### Example C-1: 4-[3-fluoro-4-(2,2,2-trichloroacetylamino)phenoxy]pyridine-2-carboxylic acid amide

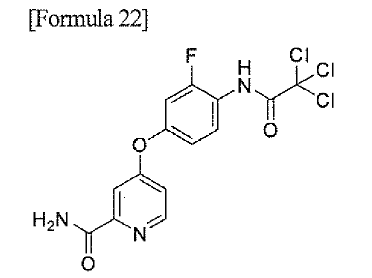

Triethylamine (246 mg) was added to a solution of 4-(4-amino-3-fluorophenoxy)pyridine-2-carboxamide (200 mg) in N-methyl-2-pyrrolidone (2 ml) with stirring and cooling on ice. After confirming the dissolution, trichloroacetic anhydride (117 µl) was added thereto at the same temperature, which was raised to room temperature and the mixture was stirred for 15 hours at room temperature. Subsequently, trichloroacetic anhydride (39 µl) was again added to the reaction mixture and the mixture was stirred at room temperature for 1 hour. Further, trichloroacetic anhydride (39 µl) was added to the reaction mixture and the mixture was stirred at room temperature for 1 hour. The reaction mixture was diluted using ethyl acetate (2 ml), the mixture was washed with water to partition the layers. Subsequently, the organic layer was washed with 5% aqueous solution of sodium chloride and dried over the magnesium sulfate. The solvent was distilled off under reduced pressure to give the residue. The residue was purified by silica gel column chromatography (Silica gel, eluent; 20 to 40/80 to 60 = ethyl acetale/n-heptane) to give the titled compound (155 mg).
¹H-NMR Spectrum (DMSO-d₆) δ (ppm): 7.12-7.17 (1H, m), 7.26 (1H, dd, J=2.4, 5.6 Hz), 7.40 (1H, dd, J=2.8,11.2 Hz), 7.45 (1H, d, J=2.4 Hz), 7.55 (1H, t, J=9.6 Hz), 7.73 (1H, brs), 8.15 (1H, brs), 8.57 (1H, d, J=5.6 Hz), 10.82 (1H, brs).

### Example C-2: N-[4-(2-aminopyridin-4-yloxy)-2-fluorophenyl]-2,2,2-trichloroacetamide

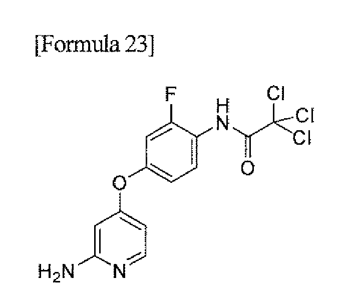

4-[3-Fluoro-4-(2,2,2-trichloroacetylamino)phenoxy]pyridine-2-carboxylic acid amide (145 mg) as a raw material was reacted and purified in the same manner as in Example A-2 to give the titled compound (91 mg).
¹H-NMR Spectrum (DMSO-d₆) δ (ppm): 5.92 (1H, d, J=2.0 Hz), 6.00-6.06 (2H, m), 6.19 (1H, dd, J=2.4, 6.0 Hz), 7.00-7.05 (1H, m), 7.24 (1H, dd, J=2.4, 11.2 Hz), 7.46 (1H, t, J=8.8 Hz), 7.85 (1H, d, J=5.6 Hz), 10.80 (1H, brs).

### Example C-3: 4-(4-(4-methylpiperazin-1-yl)-piperidine-1-carboxylic acid {4-[3-fluoro-4-(2,2,2-trichloroacetylamino)phenoxy]pyridin-2-yl]amide

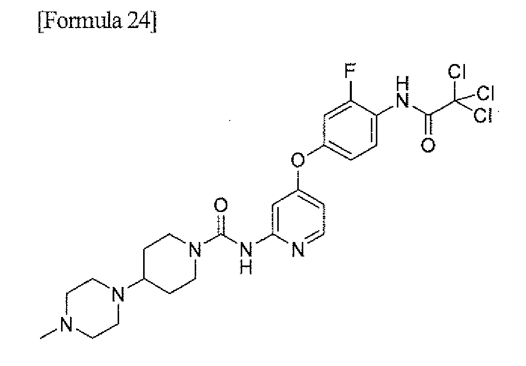

N-[4-(2-Aminopiridin-4-yloxy)-2-fluorophenyl]-2,2,2-trichloroacetamide (91 mg) as a raw material was reacted and purified in the same manner as in Example A-3 to give the titled compound (92 mg).
¹H-NMR Spectrum (DMSO-d₆) δ (ppm): 1.20-1.35 (2H, m), 1.68-1.78 (2H, m), 2.13 (3H, s), 2.23-2.50 (8H, m) 2.74 (2H, t, J=11.6 Hz), 3.33 (1H, brs), 4.11 (2H, d, J=13.2 Hz), 6.64 (1H, dd, J=2.4, 5.6 Hz), 7.02-7.07 (1H, m), 7.27 (1H, dd, J=2.8, 11.2 Hz), 7.44 (1H, d, J=2.4 Hz), 7.50 (1H, t, J=8.8 Hz), 8.16 (1H, d, J=5.6 Hz), 9.24 (1H, brs).

### Example C-4: 4-(4-methylpiperazin-1-yl)-piperidine-1-carboxylic acid [4-(4-amino-3-fluorophenoxy)-pyridin-2-yl]amide

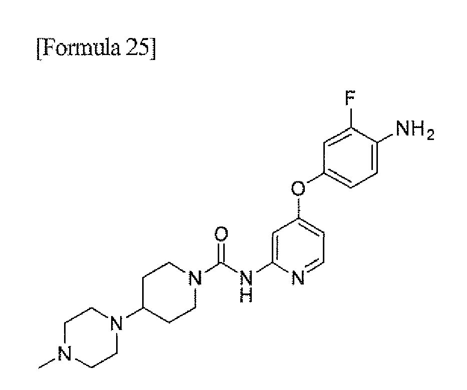

4-(4-(4-Methypiperadin-1-yl)piperidine-1-carboxylic acid {4-[3-fluoro-4-(2,2,2-trichloroacetylamino)phenoxy]pyridin-2-yl]amide (86 mg) as a raw material was reacted in the same manner as in Example B-4 to give the titled compound (48 mg).
The ¹H-NMR data of this sample corresponded to the data described in Example A-4.

### Industrial Applicability

The method for producing a phenoxypyridine derivative according to the present invention can provide a phenoxypyridine derivative useful as anti-tumor agents, angiogenesis inhibitors or inhibitors for cancer metastasis against various kinds of tumors such as pancreatic cancers, gastric cancers, colorectal cancers, breast cancers, prostate cancers, lung cancers, renal cancers, brain tumors, ovarian cancers, esophagus cancers or the like.

## Claims

1. A method for producing a compound represented by the formula (I) 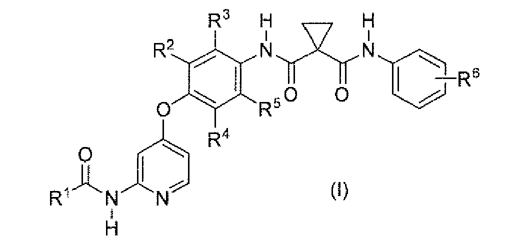 wherein R¹ represents a 4-(4-methylpiperadin-1-yl)piperidin-1-yl group or 3-hydroxyazetidin-1-yl group, R², R³, R⁴ and R⁵ may be the same or different and each represents a hydrogen atom or a fluorine atom, provided that two or three of R², R³, R⁴ and R⁵ are a hydrogen atom, and R⁶ represents a hydrogen atom or a fluorine atom,
the method comprising:
1) protecting an amino group of a compound represented by the formula (IX) 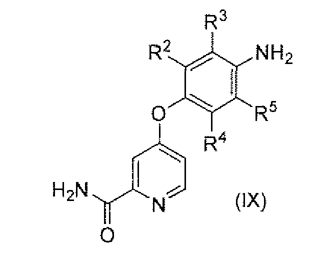 wherein R², R³, R⁴ and R⁵ have the same definitions as defined above
to produce a compound represented by the formula (VIII) 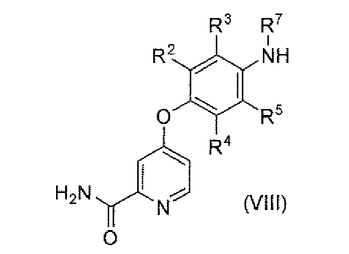 wherein R², R³, R⁴ and R⁵ have the same definitions as defined above and R⁷ represents a protection group for the amino group,
2) subsequently reacting the compound represented by the formula (VIII) with a Hofmann rearrangement agent to produce a compound represented by the formula (VI) 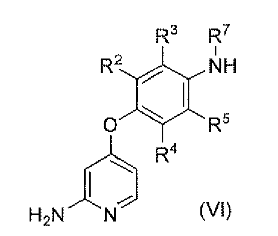 wherein R², R³, R⁴, R⁵ and R⁷ have the same definitions as defined above,
3) subsequently reacting the compound represented by the formula (VI) with a compound represented by the formula (VII) 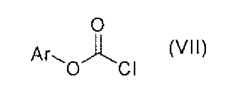 wherein Ar represents a phenyl group optionally substituted with one or two substituents selected from a halogen atom, a methyl group, a methoxy group, a nitro group, a cyano group and a trifluoromethyl group
to produce a compound represented by the formula (V) 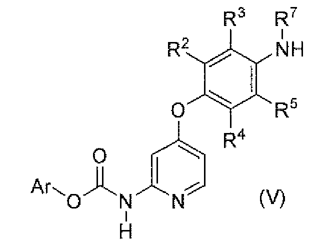 wherein R², R³, R⁴, R⁵, R⁷ and Ar have the same definitions as defined above,
4) subsequently reacting the compound represented by the formula (V) with an amine selected from 1-methyl-4-(piperidin-4-yl)piperazine and 3-hydroxyazetidine or a salt thereof to produce a compound represented by the formula (IV) or a salt thereof 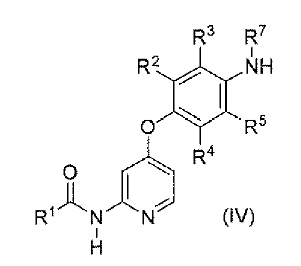 wherein R¹, R², R³, R⁴, R⁵ and R⁷ have the same definitions as defined above,
5) subsequently deprotecting the amino group of the compound represented by the formula (IV) or a salt thereof to produce a compound represented by the formula (II) or a salt thereof 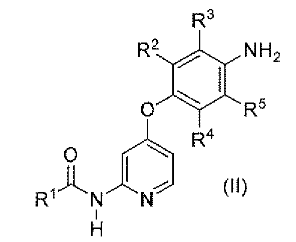 wherein R¹, R², R³, R⁴ and R⁵ have the same definitions as defined above, and
6) reacting the compound represented by the formula (II) or a salt thereof with a compound represented by the formula (III) in the presence of a condensation agent, 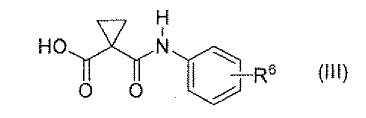
wherein R⁶ has the same definitions as defined above to produce the compound represented by the formula (I).

2. The production method according to claim 1, wherein the Hofmann rearrangement agent is iodobenzene diacetate or iodobenzene bis(trifluoroacetate).

3. The production method according to claim 1, wherein the condensation agent is O-(7-azabenzotriazol-1-yl)-N,N,N',N'-tetramethyluronium hexafluorophosphate (HATU).

4. The production method according to claim 1, wherein R⁷ is a t-butoxycarbonyl group, a trifluoroacetyl group or trichloroacetyl group.
